# FASCICULE DE BREVET EUROPEEN

(11) **EP 1 161 687 B1**
(45) Date de publication et mention de la délivrance du brevet: **07.11.2007**
(21) Numéro de dépôt: 00910938.0
(22) Date de dépôt: 14.03.2000
(51) Int. Cl.: G01N 33/74, G01N 33/566, G01N 33/573

(54) **Famille des protéines Grb7 et le récepteur de l'insuline pour le criblage de nouveaux médicaments.**
Grb7 Familie Proteinen und der Insulinrezeptor zur Screening von neuer Arzneimittel
Grb7 family proteins and the insulin receptor for screening novel medicines.

(30) Priorité: 15.03.1999 FR 9903159
(43) Date de publication de la demande: 12.12.2001
(73) Titulaire: CENTRE NATIONAL DE LA RECHERCHE SCIENTIFIQUE (CNRS), 75794 Paris Cedex 16 (FR)
(72) Inventeur: BURNOL, Anne-Françoise, F-92310 Sevres (FR); PERDEREAU, Dominique, F-92130 Issy Les Moulineaux (FR); KASUS-JACOBI, Anne, F-78180 Montigny Le Bretonneux (FR); BEREZIAT, Véronique, F-91120 Palaiseau (FR); GIRARD, Jean, F-75013 Paris (FR)
(74) Mandataire: Goulard, Sophie
(86) Numéro de dépôt international: PCT/FR2000/000613
(87) Numéro de publication internationale: WO 2000/055634

(56) Documents cités:
- WO-A-98/01475
- US-A- 5 726 027
- US-A- 5 840 536
- US-A- 5 889 150
- A KASUS-JACOBI, D PERDEREAU, C AUZAN, E CLAUSER, E VAN OBBERGHEN, F MAUVAIS-JARVIS, J GIRARD, A-F BURNOL: "Identification of the Rat Adapter Grb14 as an Inhibitor of Insulin Actions" JOURNAL OF BIOLOGICAL CHEMISTRY, vol. 273, no. 40, 2 octobre 1998 (1998-10-02), pages 26026-26035, XP002124253 cité dans la demande
- T J O'NEILL, D W ROSE, T S PILLAY, K HOTTA, J M OLEFSKY, T A GUSTAFSON: "Interaction of a GRB-IR Splice Variant (a Human GRB10 Homolog) with the Insulin and Insulin-like Growth Factor I Receptors" JOURNAL OF BIOLOGICAL CHEMISTRY, vol. 271, no. 37, 13 septembre 1996 (1996-09-13), pages 22506-22513, XP002124254 cité dans la demande
- R J DALY, G M SANDERSON, P W JANES, R L SUTHERLAND: "Cloning and Characterization of GRB14, a Novel Member of the GRB7 Gene Family" JOURNAL OF BIOLOGICAL CHEMISTRY, vol. 271, no. 21, 24 mai 1996 (1996-05-24), pages 12502-12510, XP002124255 cité dans la demande
- W HE, D W ROSE, J M OLEFSKY, T A GUSTAFSON: "Grb10 Interacts Differentially with the Insulin Receptor, Insulin-like Growth Factor I Receptor, and Epidermal Growth Factor Receptor via the Grb10 Src Homology (SH2) Domain and a Second Novel Domain Located between the Pleckstrin Homology and SH2 Domains" JOURNAL OF BIOLOGICAL CHEMISTRY, vol. 273, no. 12, 20 mars 1998 (1998-03-20), pages 6860-6867, XP002124256 cité dans la demande
- J D FRANTZ, S GIORGETTI-PERALDI, E A OTTINGER, S E SHOELSON: "Human GRB-IRbeta/GRB10" JOURNAL OF BIOLOGICAL CHEMISTRY, vol. 272, no. 5, 31 janvier 1997 (1997-01-31), pages 2659-2667, XP002124257 cité dans la demande
- F LIU, R A ROTH: "Grb-IR: A SH2-domain-containing protein that binds to the insulin receptor and inhibits its function" PROCEEDINGS OF THE NATIONAL ACADEMY OF SCIENCES OF USA, vol. 92, octobre 1995 (1995-10), pages 10287-10291, XP002124258 cité dans la demande

## Description

La présente invention se rapporte à l'utilisation de la protéine Grb14 et des protéines adaptatrices homologues (protéines de la famille Grb7), comme outil de criblage de molécules destinées au traitement des maladies impliquant l'insuline.

L'insuline, hormone principale de la régulation du métabolisme énergétique est la seule hormone hypoglycémiante de l'organisme ; elle stimule le transport du glucose et son utilisation par les tissus périphériques (muscles squelettiques et tissu adipeux) et inhibe la production endogène de glucose par le foie.

L'insuline agit par l'intermédiaire d'un récepteur qui est exprimé à la membrane plasmique des cellules. Ce récepteur fait partie de la famille des récepteurs à activité tyrosine kinase, qui sont caractérisés par la présence d'un domaine intracellulaire portant l'activité catalytique. La liaison du ligand induit la dimérisation des récepteurs, l'activation du domaine tyrosine kinase, et la phosphorylation (autophosphorylation et transphosphorylation) de résidus tyrosines spécifiques présents dans la partie cytosolique des récepteurs (Ullrich, A. et al. (1990) Cell, 61, 203-212).

Le récepteur de l'insuline possède la particularité d'être présent sous une forme naturellement dimérisée. La liaison de l'insuline à la sous-unité α extracellulaire induit des modifications conformationnelles qui aboutissent à l'activation du domaine kinase porté par la sous-unité β du récepteur, et à son autophosphorylation, nécessaire à l'activation complète du récepteur. Le récepteur de l'insuline ainsi activé phosphoryle des protéines intracellulaires qui servent d'effecteurs du signal de l'insuline.

En effet, la transduction d'un signal à l'intérieur de la cellule après la stimulation d'un récepteur à activité tyrosine kinase, fait appel à des cascades d'interactions protéine-protéine, qui aboutissent à un effet métabolique ou mitogénique, et dans lesquelles les adaptateurs moléculaires ont un rôle privilégié. Par l'intermédiaire de leurs domaines d'interactions protéiques, les adaptateurs permettent le recrutement des effecteurs successifs, constituant les voies de signalisation.

Parmi les différents relais entre le récepteur de l'insuline et ses effecteurs intracellulaires, les protéines adaptatrices les mieux caractérisées sont IRS-1, IRS-2 (*Insulin Receptor substrate-1 and* 2) et Shc (*Src and collagen homologous protein*) (White M.F. et al. (1994) J. Biol. Chem., 269, 1-4 ; Waters S.B. et al. (1996) Trends Cell Biol., 6, 1-4). Elles ne sont pas spécifiques des tissus sensibles à l'insuline et sont également phosphorylées aussi bien après l'activation d'autres récepteurs à tyrosine kinase qu'après celle de récepteurs de cytokines ou de récepteurs couplés à la protéine G (Bonfini L. et al. (1996) Trends Biochem. Sci., 21, 257-261 ; Souza S.C. et al. (1994) J. Biol. Chem., 269, 30085-30088 ; Argetsinger L.S.et al. (1995) J. Biol. Chem., 270, 14685-14692 ; Platanias L.C. et al. (1996) J. Biol. Chem., 271, 278-282 ; Velloso L.A. et al. (1996) Proc. Natl. Acad. Sci. USA, 93, 12490-12495 ; Kowalski-Chauvel A. et al. (1996) J. Biol. Chem., 271, 26536-26361).

Ainsi par exemple, la protéine Shc se lie au récepteur de l'insuline activé, puis est phosphorylée, et recrute l'adaptateur Grb2 qui se lie au niveau de résidus phosphotyrosine de Shc, par l'intermédiaire de son domaine SH2 et se lie par un domaine SH3 sur l'échangeur nucléotidique Sos, qui lui-même va permettre l'activation de Ras (Schlessinger, J. (1993), Trends Bioch. Sci., 18, 273-275).

Récemment de nouvelles protéines adaptatrices susceptibles d'être impliquées spécifiquement dans la transduction du signal de l'insuline ont été clonées par interaction avec le récepteur de l'insuline en utilisant le système double-hybride, notamment différentes isoformes de la protéine Grb10 de l'homme et de la souris (Liu F. et al. (1995) Proc. Natl. Acad. Sci. USA, 92, 10287-10291 ; O'Neill T. J. et al. (1996) J. Biol. Chem., 271, 22506-22513 ; Frantz J.D. et al. (1997) J. Biol. Chem., 272, 2659-2667)

Plus récemment encore les Inventeurs ont cloné les protéines rGrb14 et rGrb7 chez le rat, par interaction avec le récepteur de l'insuline en utilisant le système double-hybride (Kasus-jacobi et al. (1998) J. Biol. Chem., 273, 26026-26035).

Les protéines mGrb10, hGrb10, hGrb14 et rGrb14 appartiennent à la même famille de protéines adaptatrices dont le premier membre connu est la protéine Grb7 qui se lie au récepteur de l'EGF, du Ret et du PDGF (Margolis B. (1992) Proc. Natl. Acad. Sci. USA, 89, 8894-8898). Ci-après les protéines de cette famille sont dénommées protéines de la famille Grb7.

Ces protéines qui ont été clonées par interaction avec des récepteurs activés de l'insuline, semblent jouer un rôle important dans la transduction du signal de l'insuline.

Ainsi les Inventeurs ont montré que l'expression de la protéine rGrb14 est très bien corrélée avec la sensibilité des tissus à l'insuline et que sa surexpression dans des cellules CHO-IR (*Chinese Hamster Ovary* exprimant des taux élevés de récepteurs de l'insuline d'origine humaine) inhibe les effets de l'insuline en diminuant l'activation de IRS-1 sans modifier l'autophosphorylation du récepteur de l'insuline. (Kasus-Jacobi et al. (1998) déjà cité).

Les protéines adaptatrices de la famille de Grb7 sont caractérisées par la succession de trois domaines :
- une séquence riche en proline appelée PP, près de l'extrémité amino-terminale,
- un domaine central appelé PH (*Pleckstrin homology*) et
- un domaine appelé SH2 (*Src homology* 2) à l'extrémité carboxy-terminale, connu pour interagir avec les séquences contenant des phosphotyrosines (Ooi J. et al. (1995) Oncogene, 10, 1621-1630 ; Margolis B. (1992) déjà cité ; Daly R.J. (1996) déjà cité).

Outre ces domaines qui ont été déjà bien étudiés dans d'autres protéines, les Inventeurs ont mis en évidence un nouveau domaine sur la protéine rGrb14, appelé PIR (*Phosphorylated Insulin Receptor Interacting Region*) correspondant aux résidus 340 à 437 de la protéine ; par comparaison entre les protéines Grb7, Grb10 et Grb14, les Inventeurs ont montré qu'une séquence de 43 acides aminés correspondant aux acides 365 à 407 de la protéine rGrb14 est hautement conservée dans l'ensemble de la famille de ces protéines (Kasus-Jacobi et al. (1998) déjà cité) et devrait jouer un rôle particulier dans la fixation de ces protéines sur le récepteur de l'insuline.

Le domaine PIR est homologue au domaine BPS (*Between PH and* SH2), (Kasus-Jacobi et al. (1998) déjà cité), récemment mis en évidence sur la protéine hGrb10 (He W. et al. (1998) J. Biol. Chem., 273, 6860-6867) et correspond aux acides aminés 358-434 de la protéine Grb14.

L'association entre le récepteur de l'insuline activé et les protéines de la famille Grb7 fait intervenir les deux domaines PIR et SH2. En fonction de la protéine Grb considérée, le rôle respectif des deux domaines est plus ou moins important. En effet, c'est essentiellement le PIR qui est responsable de la liaison de Grb14 sur le récepteur de l'insuline (Kasus-Jacobi et al. (1998) déjà cité), alors que le PIR et le SH2 sont impliqués dans l'interaction entre Grb10 et le récepteur (He et al (1998) déjà cité).

Plusieurs équipes ont montré qu'il y avait des défauts de phosphorylation du récepteur de l'insuline ainsi que des altérations des effets de l'insuline sur le transport de glucose et sur l'activation de certaines enzymes chez des patients obèses ou diabétiques (Amer, P. et al., J. N. (1987), Diabetologia, 30, 437-440; Caro, J. F. et al. (1987), J. Clin. Invest., 79, 1330-1337 ; Mandarino, L. J. (1989), Diab. Metab. Rev., 5, 475-486).

Des mutations du gène du récepteur de l'insuline peuvent conduire par différents mécanismes à une diminution de l'activité tyrosine kinase du récepteur, contribuant ainsi au développement d'un état de résistance à l'insuline et à l'instauration de pathologies comme l'obésité et le diabète non-insulino-dépendant (DNID) (Taylor, S. I. (1992), Diabetes, 41, 1473-1490.

Dans des états de résistance à l'insuline, il y a développement d'une hyperglycémie lorsque la sécrétion endogène d'insuline n'est plus suffisante, et il faut faire appel à une insulinothérapie pour maintenir l'homéostasie glucidique. Après 10 ans d'évolution du diabète, on observe dans 30% des cas des complications sévères. Ces complications, secondaires à un mauvais contrôle de la glycémie, ont de très sérieuses implications cliniques (insuffisances rénales, nécrose et amputation des membres inférieurs, cécité) qui conduisent à un raccourcissement de l'espérance de vie des patients.

La normalisation de l'activité tyrosine kinase lorsqu'elle est perturbée peut être envisagée soit directement en utilisant des molécules qui agissent sur cette enzyme (Levitzki et al. (1995), Science 267, 1782-1788), soit indirectement en inhibant les interactions entre les protéines adaptatrices et la tyrosine kinase (Pendergast et al. (1993), Cell, 75, 175-185).

Or, les Inventeurs ont montré, de manière surprenante, que la liaison au récepteur de l'insuline activé, du domaine PIR des protéines de la famille des protéines Grb7 (Grb14, Grb10, et Grb7), seul ou associé au fragment SH2 (PIR-SH2), inhibe l'activité tyrosine kinase dudit récepteur.

La présente invention a pour objet l'utilisation d'un fragment constitué par le domaine PIR d'une protéine de la famille des protéines Grb7, comme outil de criblage de molécules aptes à moduler l'activité tyrosine kinase du récepteur de l'insuline, destinées au traitement des maladies impliquant l'insuline.

Selon un mode de réalisation avantageux de ladite utilisation, ledit fragment est sélectionné dans le groupe constitué par les séquences : SEQ ID NO:1 2, 5, 6, 9, 10, 13, 14, 17, 18, 21, 22, 25 et 26 qui correspondent à des fragments PIR (résidus 365-407 et résidus 353-436) des protéines rGrb14, hGrb14, mGrb10, hGrb10, rGrb7, hGrb7 et mGrb7).

Au sens de la présente invention la numérotation des résidus des fragments de protéines est donnée en référence à la séquence de la protéine rGrb14, après alignement.

De façon intéressante, les Inventeurs ont montré que l'effet inhibiteur de la protéine Grb14 est reproduit par les protéines de fusion GST-PIR et GST-PIR+SH2 purifiées, obtenues par fusion de la GST avec le domaine PIR ou le domaine PIR+SH2 de rGrb14. En revanche cet effet inhibiteur n'est pas observé avec la protéine de fusion GST-SH2 obtenue par fusion GST avec le domaine SH2 de rGrb14.

De façon inattendue, les Inventeurs ont montré que le domaine PIR seul a une activité équivalente à celle de la protéine entière alors que le domaine PIR+SH2 a un effet inhibiteur beaucoup plus important que le PIR exprimé seul. En effet l'inhibition totale de l'activité tyrosine kinase des récepteurs de l'insuline est obtenue lorsque l'on ajoute 0,3 µg de protéine GST-PIR, alors qu'il ne faut que 0,03 µg de GST-PIR+SH2. Il semble donc que si le domaine SH2 n'a pas d'activité inhibitrice propre, par contre il potentialise fortement l'effet du PIR.

De façon comparable, les Inventeurs ont montré que les domaines PIR et PIR+SH2 de Grb10 ont un effet inhibiteur sur l'activité tyrosine kinase du récepteur de l'insuline. Le domaine SH2 de Grb10 n'a pas en lui-même d'effet inhibiteur, mais il potentialise également l'inhibition induite par le PIR.

De plus, les Inventeurs ont montré que le récepteur de l'insuline est plus sensible à l'effet inhibiteur de Grb14 qu'à celui de Grb10 et de Grb7, et que l'effet peut être obtenu aussi bien avec la protéine entière qu'avec le domaine PIR ou le domaine PIR-SH2.

Les domaines PIR et PIR-SH2 des protéines Grb14, Grb10 et Grb7 se comportent donc comme des inhibiteurs endogènes de l'activité tyrosine kinase du récepteur de l'insuline, ce qui est une fonction tout à fait nouvelle pour des adaptateurs moléculaires. En effet, contrairement aux protéines adaptatrices IRS-1, IRS-2 ou Shc qui sont des intermédiaires entre le récepteur de l'insuline et des effecteurs cellulaires, lesdits domaines des protéines de la famille Grb7 agissent directement sur l'activité tyrosine kinase du récepteur de l'insuline.

En conséquence, les domaines PIR et PIR-SH2 de la protéine Grb14 et des protéines adaptatrices homologues (protéines de la famille Grb7) constituent des cibles potentielles pour des médicaments.

En effet, des composés qui sont susceptibles d'augmenter ou de supprimer les interactions des domaines desdites protéines pourraient prévenir ou guérir les troubles de l'organisme liés à une modification de l'activité de la protéine kinase du récepteur de l'insuline.

En conséquence, la présente invention se rapporte donc également à un procédé de détection in vitro de molécules aptes à stimuler ou inhiber (moduler) l'activité tyrosine kinase du récepteur de l'insuline, caractérisé en ce qu'il comprend :
a) la mise en contact du récepteur de l'insuline activé avec un fragment constitué par le domaine PIR d'une protéine de la famille des protéines Grb7, et la molécule à tester, dans des conditions permettant la formation d'une liaison entre ledit récepteur et ledit fragment,
b) l'addition d'un substrat de la tyrosine kinase,
c) la mesure de l'activité tyrosine kinase, et
d) la détermination de la modulation (inhibition ou stimulation) de l'activité tyrosine kinase par comparaison avec un contrôle constitué par le récepteur de l'insuline activé et ledit fragment.

Conformément à l'invention, ledit fragment est de préférence sélectionné dans le groupe constitué par les séquences SEQ ID NO:1 ,2, 5, 6, 9, 10, 13, 14, 17, 18, 21, 22, 25 et 26.

Selon un mode de mise en oeuvre avantageux dudit procédé, préalablement à l'étape a) ci-dessus, une présélection des molécules aptes à stimuler ou inhiber (moduler) les interactions d'un fragment constitué par le domaine PIR d'une protéine de la famille des protéines Grb7 avec le récepteur de l'insuline, est réalisée par :
1) l'immobilisation dudit fragment sur un support solide,
2) la mise en contact de la molécule à tester avec ledit fragment, puis
3) l'incubation avec le récepteur de l'insuline marqué et préalablement activé, dans des conditions permettant la formation d'une liaison entre ledit récepteur et ledit fragment,
4) la séparation du récepteur marqué non retenu sur le support,
5) la détection du complexe éventuellement formé entre ledit fragment et le récepteur de l'insuline activé, et
6) la détermination de l'effet de la molécule (inhibition ou stimulation de l'interaction fragment-récepteur), par comparaison avec un contrôle comprenant ledit fragment et le récepteur de l'insuline.

Pour permettre l'immobilisation dudit fragment sur un support solide, ledit fragment peut par exemple être exprimé en fusion avec une protéine telle que la GST.

Ledit récepteur peut par exemple être marqué avec une molécule radioactive, ou fusionné à une protéine fluorescente telle que la GFP (*Green Fluorescent Protein).*

Lorsque ledit récepteur est marqué avec une molécule fluorescente ou radioactive, l'interaction entre ledit fragment et ledit récepteur est détectée par lecture de la fluorescence ou de la radioactivité retenue sur le support solide.

Les composés ainsi sélectionnés sont potentiellement utiles pour prévenir ou traiter des maladies impliquant l'insuline comme par exemple le diabète et l'obésité ou d'autres pathologies caractérisées par une résistance à l'insuline telles que l'ovaire polycystique (Legro, R. S. et al. (1998), Rec. Progr. Hormone Res., 53, 217-255) ou le syndrome X (Komers, R. et al. (1998), Physiol. Res., 47, 215-225).

Outre les dispositions qui précèdent, l'invention comprend encore d'autres dispositions, qui ressortiront de la description qui va suivre, qui se réfère à des exemples de mise en oeuvre du procédé objet de la présente invention ainsi qu'aux dessins annexés, dans lesquels :

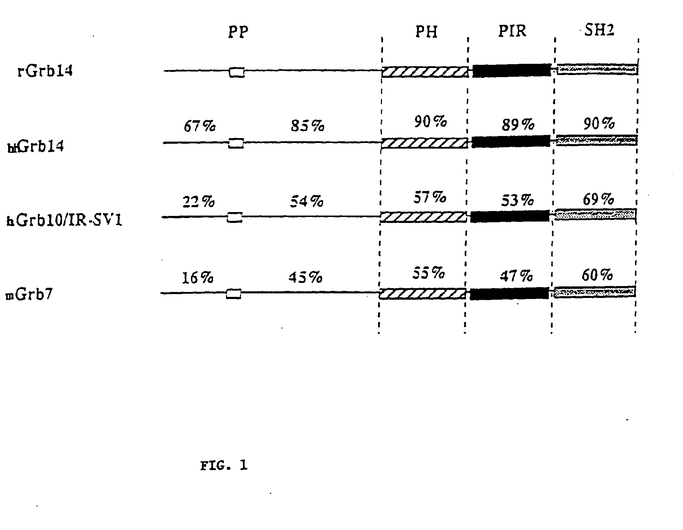

- la figure 1 illustre l'alignement des protéines de la famille des protéines Grbs. Les pourcentages d'identité en acides aminés des domaines sont exprimés par rapport au domaine homologue de rGrb14. PP : motif riche en résidus proline, site de liaison de protéines contenant des domaines SH3 ; PH : domaine d'homologie avec la plexstrine, association avec des phospholipides ou des protéines ; PIR, *phosphorylated insulin receptor interacting region ;* SH2, domaine permettant une interaction avec des résidus phosphotyrosines.

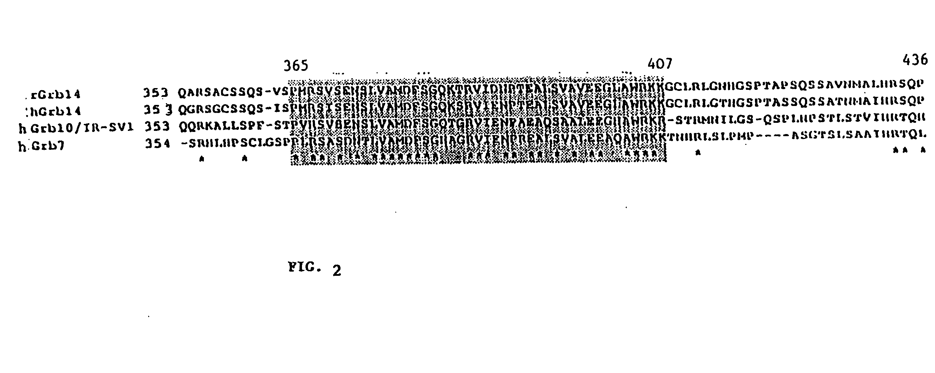

- la figure 2 illustre l'alignement des séquences des domaines PIR des protéines de la famille des protéines Grb : rGrb14, hGrb14, hGrb10 et hGrb7. La numérotation des acides aminés est donnée en référence à la séquence de la protéine rGrb14. Les acides aminés conservés sont indiqués par un astérisque. Le domaine conservé correspondant aux résidus 365-407 des protéines Grb est grisé.

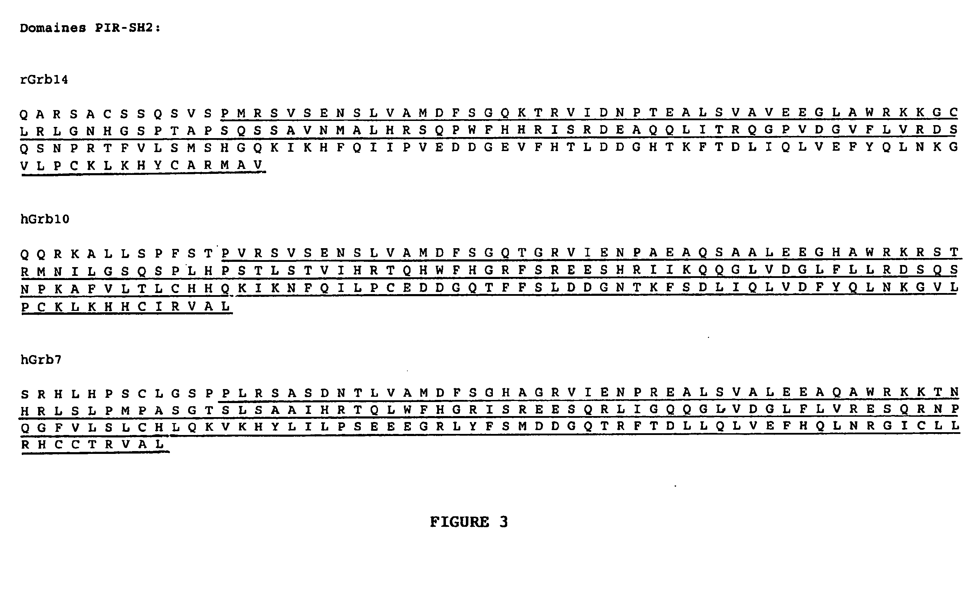

- la figure 3 illustre la séquence des domaines PIR-SH2 des protéines de la famille des protéines Grb : rGrb14, hGrb10 et hGrb7. La séquence complète du domaine PIR-SH2 (résidus 353-538) de rGrb14 (SEQ ID NO:4), hGrb10 (SEQ ID NO:16) et hGrb7 (SEQ ID NO:24) est présentée. La séquence du fragment 405-538 du domaine PIR-SH2 de rGrb14 (SEQ ID NO:3), hGrb10 (SEQ ID NO:15) et de hGrb7 (SEQ ID NO:23) est soulignée.

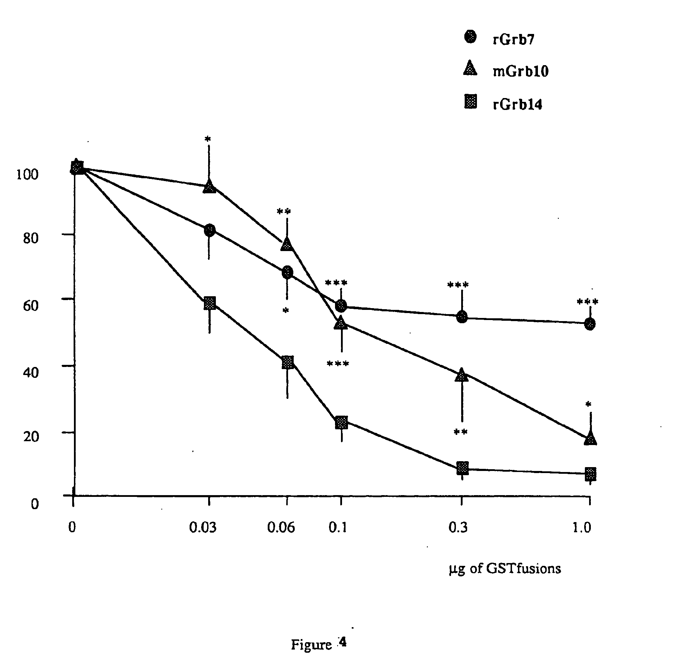

- la figure 4 illustre l'effet des protéines Grbs sur l'activité tyrosine kinase des récepteurs de l'insuline; (●) GST-rGrb14 ; (▲) GST-mGrb10 ; (■) GST-rGrb7. Les résultats sont exprimés en pourcentage de la valeur obtenue en l'absence de protéine ajoutée. Nombre d'expériences = 4. Les effets des protéines GST-mGrb10 et GST-rGrb7 comparés avec ceux de la protéine GST-rGrb14 montrent des différences statistiquement significatives, indiquées par: * pour p<0,05, ** pour p<0,01 et *** pour p<0,001.

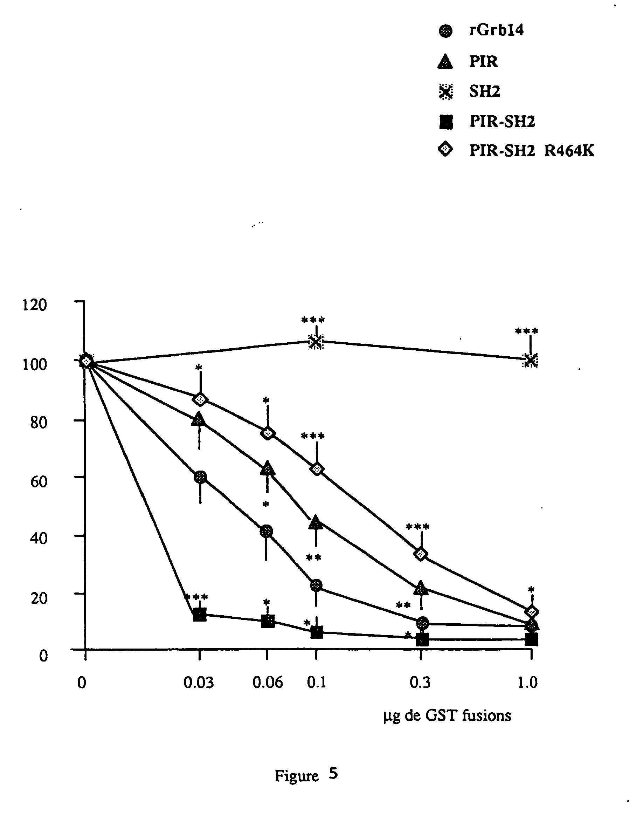

- la figure 5 illustre l'inhibition de la tyrosine kinase des récepteurs de l'insuline par la protéine rGrb14 : (●) GST-rGrb14 ; (▲) GST-PIR de rGrb14 ; ( 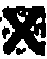 )GST-SH2 de rGrb14; (■) GST-PIR+SH2 de rGrb14 ; (◇) GST-PIR+SH2 R464K de rGrb14. Les résultats sont exprimés en pourcentage de la valeur obtenue en l'absence de protéine ajoutée. Nombre d'expériences = 5. Les effets des différentes constructions de rGrb14 comparés avec ceux de la protéine GST-rGrb14 entière montrent des différences statistiquement significatives, indiquées par: * pour p<0,05, ** pour p<0,01 et *** pour p<0,001.

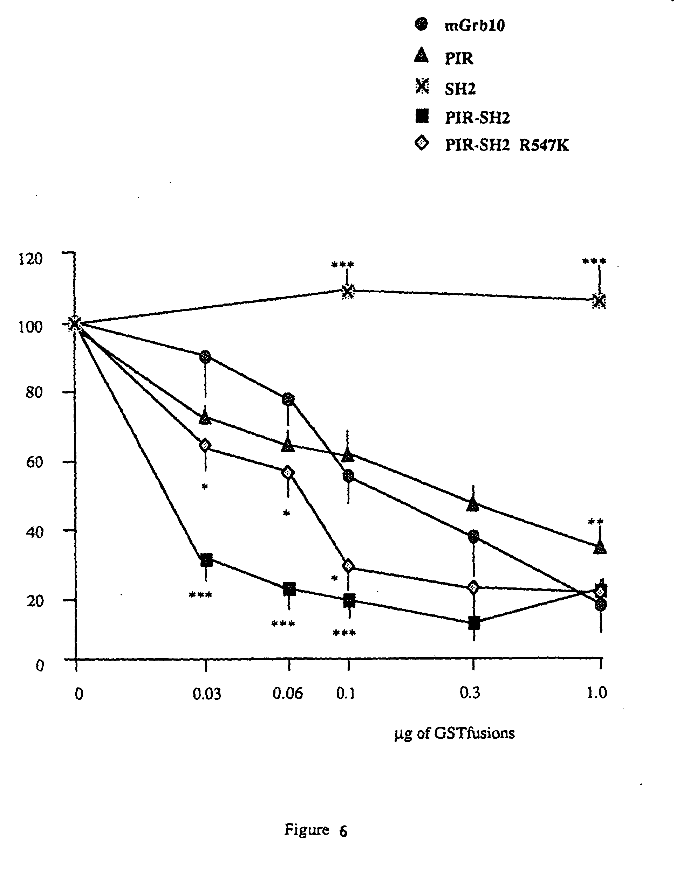

- la figure 6 illustre l'inhibition de l'activité tyrosine kinase des récepteurs de l'insuline par les différents domaines de mGrb10 : (●) GST-mGrb10 ; (▲) GST-PIR de mGrb10 ; ( 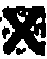 ) GST-SH2 de mGrb10 ; (■) GST-PIR+SH2 de mGrb10 ; (◇) GST-PIR+SH2 R547K de mGrb10. Les résultats sont exprimés en pourcentage de la valeur obtenue en l'absence de protéine ajoutée. Nombre d'expériences = 4. Les effets des différentes constructions de mGrb10 comparés avec ceux de la protéine GST-mGrb10 entière montrent des différences statistiquement significatives, indiquées par: * pour p<0,05, ** pour p<0,01 et *** pour p<0,001.

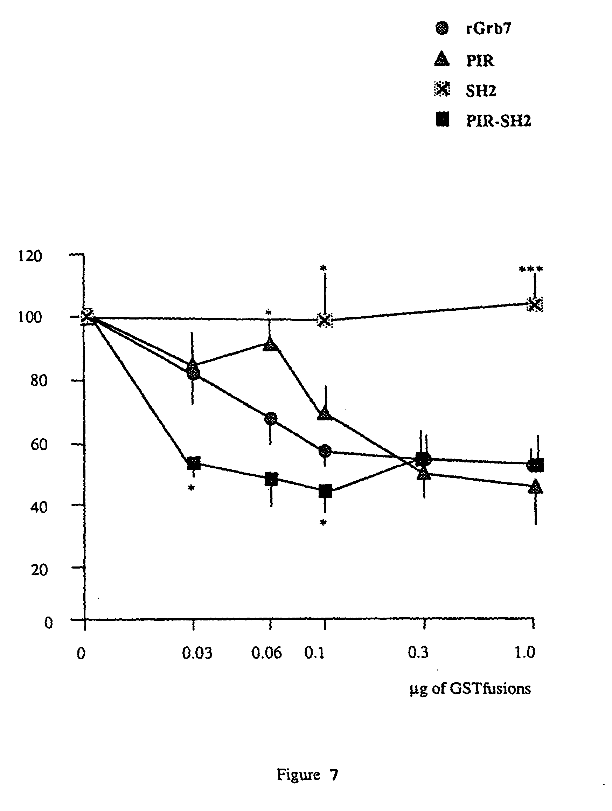

- la figure 7 illustre l'inhibition de l'activité tyrosine kinase des récepteurs de l'insuline par les différents domaines de rGrb7: (●) GST-rGrb7; (▲) GST-PIR de rGrb7; ( 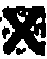 ) GST-SH2 de rGrb7 ; (■) GST-PIR+SH2 de rGrb7. Les résultats sont exprimés en pourcentage de la valeur obtenue en absence de protéine ajoutée. Nombre d'expériences= 2. Les effets des différentes constructions de rGrb7 comparés avec ceux de la protéine GST-rGrb7 entière montrent des différences statistiquement significatives, indiquées par: * pour p<0,05, ** pour p<0,01 et *** pour p<0,001.

### Exemple 1 : Comparaison de l'effet des protéines rGrb14, mGrb10 et rGrb7 sur l'activité tyrosine kinase des récepteurs de l'insuline

### 1. Mode opératoire:

Des récepteurs de l'insuline sont partiellement purifiés à partir de cellules CHO-IR, en passant un lysat cellulaire sur une colonne de lectine de germe de blé et en éluant les glycoprotéines retenues avec de la N-acétylglucosamine 0,3M. Les récepteurs de l'insuline ainsi purifiés sont incubés en présence d'insuline (0 ou 10⁻⁷ M) pendant 1 heure à température ambiante. On ajoute alors un tampon contenant de l'ATP 20 µM, des ions MnCl₂ et MgCl, et du [γ-³²P] ATP pour permettre aux récepteurs de s'autophosphoryler, et des quantités croissantes des protéines Grbs purifiées exprimées en fusion avec la GST. 30 minutes après, on ajoute 15 µg d'un substrat de synthèse, le poly Glu-Tyr (4:1). L'activité tyrosine kinase des récepteurs est mesurée par l'incorporation de radioactivité dans le poly Glu-Tyr pendant 30 min.

### 2. Résultats:

Ils sont représentés à la figure 4.

L'addition des protéines de fusion GST-rGrb14 et GST-mGrb10 induit une inhibition dose-dépendante de l'activité tyrosine kinase des récepteurs de l'insuline, et les concentrations les plus élevées permettent une inhibition totale de l'enzyme. Par comparaison, la protéine GST-rGrb7 ne permet au maximum qu'une inhibition de 40%. La courbe dose-réponse de l'effet de GST-mGrb10 est déplacée sur la droite par rapport à la courbe de l'effet de GST-rGrb14. Une inhibition de 50% de l'activité tyrosine kinase des récepteurs est obtenue en utilisant respectivement 0,04 µg de GST-rGrb14 et 0,13 µg de GST-mGrb10. L'activité tyrosine kinase des récepteurs de l'insuline est donc plus sensible à l'effet inhibiteur de rGrb14 qu'à celui de mGrb10.

Ces résultats montrent que les protéines Grbs ont une activité inhibitrice sur l'activité tyrosine kinase des récepteurs de l'insuline, et que la protéine rGrb14 a l'effet inhibiteur le plus important.

### Exemple 2 : Effet inhibiteur des différents domaines de rGrb14 sur l'activité tyrosine kinase des récepteurs de l'insuline

### 1. Mode opératoire:

Les récepteurs de l'insuline sont partiellement purifiés comme décrit dans l'exemple 1. Les différents domaines de rGrb14 (rGrb14, PIR, SH2, PIR+SH2, PIR+SH2 R464K) sont produits en fusion avec la GST et purifiés. L'effet inhibiteur de ces protéines sur l'activité tyrosine kinase des récepteurs de l'insuline est analysé comme décrit dans l'exemple 1.

### 2. Résultats:

Ils sont représentés à la figure 5.

Le domaine PIR exerce un effet inhibiteur comparable à celui de la protéine rGrb14 entière, alors que le domaine SH2 n'a aucun effet (de même que la protéine délétée des régions PIR+SH2, résultats non montrés). Cependant, le domaine PIR+SH2 a un effet inhibiteur beaucoup plus important que le PIR seul ou la protéine entière (l'effet inhibiteur maximal est obtenu en ajoutant 0,03 µg de protéine). Cette potentialisation est supprimée par la mutation du résidu arginine 464 du motif FLVRES conservé qui inactive le domaine SH2, puisque le domaine PIR+SH2 R464K a le même effet que le PIR seul.

Ces résultats montrent que l'effet inhibiteur de rGrb14 sur l'activité tyrosine kinase des récepteurs de l'insuline est due à la présence du PIR. Le domaine SH2 seul n'a aucun effet, mais il potentialise l'effet du PIR.

### Exemple 3 : Effet inhibiteur des différents domaines de mGrb10 sur l'activité tyrosine kinase des récepteurs de l'insuline

### 1. Mode opératoire:

Le mode opératoire est identique à celui décrit dans l'exemple 2.

### 2. Résultats:

Ils sont représentés à la figure 6.

Le domaine PIR exerce un effet inhibiteur comparable à celui exercé par la protéine entière . Le domaine SH2 seul n'a pas d'action inhibitrice, mais il potentialise l'inhibition exercée par le PIR (la courbe dose-réponse du PIR+SH2 est décalée vers la gauche). La mutation du résidu arginine du motif FLLRDS du domaine SH2 inhibe l'effet potentialisateur du domaine PIR+SH2 (mutant PIR+SH2 R547K).

Ces résultats montrent que les domaines PIR ou PIR-SH2 de rGrb14 ont un effet inhibiteur plus important que les domaines PIR ou PIR-SH2 de mGrb10.

### Exemple 4 : Effet inhibiteur des différents domaines de rGrb7 sur l'activité tyrosine kinase des récepteurs de l'insuline

### 1. Mode opératoire:

Le mode opératoire est identique à celui décrit à l'exemple 2.

### 2. Résultats:

Ils sont représentés à la figure 7.

Le domaine PIR exerce un effet inhibiteur comparable à celui exercé par la protéine entière. Le domaine SH2 seul n'a pas d'action inhibitrice, mais il potentialise l'inhibition exercée par le PIR (la courbe dose-réponse du PIR+SH2 est décalée vers la gauche). Les domaines PIR et PIR-SH2 de rGrb7 ont un effet inhibiteur moindre comparé à celui des domaines PIR et PIR-SH2 des protéines rGrb14 et mGrb10.

## Revendications

1. Utilisation d'un fragment constitué par le domaine PIR d'une protéine de la famille des protéines Grb7, comme outil de criblage de molécules aptes à moduler l'activité tyrosine kinase du récepteur de l'insuline, destinées au traitement des maladies impliquant l'insuline.

2. Utilisation selon la revendication 1, **caractérisée en ce que** ledit fragment est sélectionné dans le groupe constitué par les séquences SEQ ID NO:1, 2, 5, 6, 9, 10, 13, 14, 17, 18, 21, 22, 25 et 26.

3. Procédé de détection *in vitro* de molécules aptes à moduler l'activité tyrosine kinase du récepteur de l'insuline, **caractérisé en ce qu'**il comprend :
a) la mise en contact d'un récepteur de l'insuline activé avec un fragment constitué par le domaine PIR d'une protéine de la famille des protéines Grb7, et la molécule à tester, dans des conditions permettant la formation d'une liaison entre ledit récepteur et ledit fragment,
b) l'addition d'un substrat de la tyrosine kinase,
c) la mesure de l'activité tyrosine kinase, et
d) la détermination de la modulation de l'activité tyrosine kinase par comparaison avec un contrôle constitué par le récepteur de l'insuline activé et ledit fragment.

4. Procédé selon la revendication 3, **caractérisé en ce que** ledit fragment est sélectionné dans le groupe constitué par les SEQ ID NO:1, 2, 5, 6, 9, 10, 13, 14, 17, 18, 21, 22, 25 et 26.

5. Procédé selon la revendication 3 ou la revendication 4, **caractérisé en ce que** préalablement à l'étape a), une présélection des molécules aptes à moduler les interactions d'un fragment constitué par le domaine PIR d'une protéine de la famille des protéines Grb7 avec le récepteur de l'insuline, est réalisée par :
1) l'immobilisation dudit fragment sur un support solide,
2) la mise en contact de la molécule à tester avec ledit fragment, puis
3) l'incubation avec le récepteur de l'insuline marqué et préalablement activé, dans des conditions permettant la formation d'une liaison entre ledit récepteur et ledit fragment,
4) la séparation du récepteur marqué non retenu sur le support,
5) la détection du complexe éventuellement formé entre ledit fragment et le récepteur de l'insuline activé et
6) la détermination de l'effet de la molécule, inhibition ou stimulation de l'interaction fragment-récepteur par comparaison avec un contrôle comprenant ledit fragment et le récepteur de l'insuline.

## Claims

1. Use of a fragment consisting of the PIR domain of a protein from the Grb7 protein family, as a screening tool for molecules capable of modulating the tyrosine kinase activity of the insulin receptor, intended for the treatment of diseases that involve insulin.

2. Use according to claim 1, **characterised in that** said fragment is selected from the group consisting of the sequences SEQ ID NO:1, 2, 5, 6, 9, 10, 13, 14, 17, 18, 21, 22, 25 and 26.

3. Method of *in vitro* detection of molecules capable of modulating the tyrosine kinase activity of the insulin receptor, **characterised in that** it comprises:
a) bringing an insulin receptor activated with a fragment consisting of the PIR domain of a protein of the Grb7 protein family into contact with the molecule which is to be tested, under conditions that allow the formation of a bond between said receptor and said fragment,
b) adding a tyrosine kinase substrate,
c) measuring the tyrosine kinase activity, and
d) determining the modulation of the tyrosine kinase activity by comparison with a control consisting of the activated insulin receptor and said fragment.

4. Method according to claim 3, **characterised in that** said fragment is selected from the group consisting of the sequences SEQ ID NO:1, 2, 5, 6, 9, 10, 13, 14, 17, 18, 21, 22, 25 and 26.

5. Method according to claim 3 or 4, **characterised in that** prior to step a) a preselection of the molecules capable of modulating the interactions of a fragment consisting of the PIR domain of a protein from the Grb7 protein family with the insulin receptor is carried out by:
1) immobilising said fragment on a solid support,
2) bringing the molecule which is to be tested into contact with said fragment, then
3) incubating with labelled and previously activated insulin receptor, under conditions that allow a bond to be formed between said receptor and said fragment,
4) separating the labelled receptor not retained on the support,
5) detecting any complex which may have been formed between said fragment and the activated insulin receptor and
6) determining the effect of the molecule, inhibition or stimulation of the fragment/receptor interaction by comparison with a control comprising said fragment and the insulin receptor.

## Patentansprüche

1. Verwendung eines aus dem PIR-Bereich eines Proteins aus der Familie der Grb7-Proteine bestehenden Fragmentes als Hilfsmittel zum Screening von Molekülen, die dazu geeignet sind, die Tyrosinkinaseaktivität des Insulinrezeptors zu modulieren, und zur Behandlung von Erkrankungen bestimmt sind, die mit Insulin im Zusammenhang stehen.

2. Verwendung nach Anspruch 1, **dadurch gekennzeichnet, dass** das Fragment aus der Gruppe bestehend aus den Sequenzen SEQ ID NO: 1, 2, 5, 6, 9, 10, 13, 14, 17, 18, 21, 22, 25 und 26 ausgewählt ist.

3. Verfahren zur in vitro-Detektion von Molekülen, die dazu geeignet sind, die Tyrosinkinaseaktivität des Insulinrezeptors zu modulieren, **dadurch gekennzeichnet, dass** es umfasst:
a) Inkontaktbringen eines aktivierten Insulinrezeptors mit einem aus dem PIR-Bereich eines Proteins aus der Familie der Grb7-Proteine bestehenden Fragment und dem zu testenden Molekül unter Bedingungen, welche die Bildung einer Bindung zwischen dem Rezeptor und dem Fragment ermöglichen,
b) Zugabe eines Tyrosinkinase-Substrates,
c) Messung der Tyrosinkinaseaktivität, und
d) Bestimmung der Modulation der Tyrosinkinaseaktivität durch Vergleich mit einer Kontrolle, bestehend aus dem aktivierten Insulinrezeptor und dem Fragment.

4. Verfahren nach Anspruch 3, **dadurch gekennzeichnet, dass** das Fragment aus der Gruppe bestehend aus den SEQ ID NO: 1, 2, 5, 6, 9, 10, 13, 14, 17, 18, 21, 22, 25 und 26 ausgewählt ist.

5. Verfahren nach Anspruch 3 oder Anspruch 4, **dadurch gekennzeichnet, dass** vor dem Schritt a) eine Vorauswahl der Moleküle durchgeführt wird, die geeignet sind, die Wechselwirkungen eines aus dem PIR-Bereich eines Proteins aus der Familie der Grb7-Proteine bestehenden Fragmentes mit dem Insulinrezeptor zu modulieren, und zwar durch:
1) Immobilisieren des Fragmentes auf einem festen Träger,
2) Inkontaktbringen des zu testenden Moleküls mit dem Fragment, daraufhin
3) Inkubation mit dem markierten und vorausgehend aktivierten Insulinrezeptor unter Bedingungen, welche die Bildung einer Bindung zwischen dem Rezeptor und dem Fragment ermöglichen,
4) Abtrennen des markierten Rezeptors, der nicht auf dem Träger zurückgehalten wurde,
5) Detektieren des schließlich zwischen dem Fragment und dem aktivierten Insulinrezeptor gebildeten Komplexes, und
6) Bestimmen des Effektes des Moleküls, Inhibieren oder Stimulieren der Wechselwirkung zwischen Fragment und Rezeptor durch Vergleich mit einer Kontrolle, welche dieses Fragment und den Insulinrezeptor umfasst.


### LISTE DE SEQUENCES

<110> BURNOL, ANNE-FRANCOISE
   PERDEREAU, DOMINIQUE
   KASUS-JACOBI, ANNE
   BEREZIAT, VERONIQUE
   GIRARD, JEAN
   CENTRE NATIONAL DE LA RECHERCHE SCIENTIFIQUE
<120> UTILISATION DE LA PROTEINE Grb14 ET DES PROTEINES ADAPTATRICES HOMOLOGUES COMME OUTIL DE CRIBLAGE DE MOLECULES DESTINEES AU TRAITEMENT DES MALADIES IMPLIQUANT L'INSULINE
<130> 64441EXT
<140>
   <141>
<160> 28
<170> PatentIn Ver. 2.1
<210> 1
   <211> 43
   <212> PRT
   <213> Rattus sp.
<400> 1
   Pro Met Arg Ser Val Ser Glu Asn Ser Leu Val Ala Met Asp Phe Ser
    1               5                  10                  15
   Gly Gln Lys Thr Arg Val Ile Asp Asn Pro Thr Glu Ala Leu Ser Val
               20                  25                  30
   Ala Val Glu Glu Gly Leu Ala Trp Arg Lys Lys
           35                  40
<210> 2
   <211> 84
   <212> PRT
   <213> Rattus sp.
<400> 2
   Gln Ala Arg Ser Ala Cys Ser Ser Gln Ser Val Ser Pro Met Arg Ser
   1 5 10 15
   Val Ser Glu Asn Ser Leu Val Ala Met Asp Phe Ser Gly Gln Lys Thr
   20 25 30
   Arg Val Ile Asp Asn Pro Thr Glu Ala Leu Ser Val Ala Val Glu Glu
   35 40 45
   Gly Leu Ala Trp Arg Lys Lys Gly Cys Leu Arg Leu Gly Asn His Gly
   50 55 60
   Ser Pro Thr Ala Pro Ser Gln Ser Ser Ala Val Asn Met Ala Leu His
   65 70 75 80
   Arg Ser Gln Pro
<210> 3
   <211> 174
   <212> PRT
   <213> Rattus sp.
<400> 3
   Pro Met Arg Ser Val Ser Glu Asn Ser Leu Val Ala Met Asp Phe Ser
   1               5                   10                  15
   Gly Gln Lys Thr Arg Val Ile Asp Asn Pro Thr Glu Ala Leu Ser Val
               20                  25                  30
   Ala Val Glu Glu Gly Leu Ala Trp Arg Lys Lys Gly Cys Leu Arg Leu
           35                  40                  45
   Gly Asn His Gly Ser Pro Thr Ala Pro Ser Gln Ser Ser Ala Val Asn
       50                  55                  60
   Met Ala Leu His Arg Ser Gln Pro Trp Phe His His Arg Ile Ser Arg
   65                  70                  75                  80
   Asp Glu Ala Gln Gln Leu Ile Thr Arg Gln Gly Pro Val Asp Gly Val
                   85                  90                  95
   Phe Leu Val Arg Asp Ser Gln Ser Asn Pro Arg Thr Phe Val Leu Ser
               100                 105                 110
   Met Ser His Gly Gln Lys Ile Lys His Phe Gln Ile Ile Pro Val Glu
           115                 120                 125
   Asp Asp Gly Glu Val Phe His Thr Leu Asp Asp Gly His Thr Lys Phe
       130                 135                 140
   Thr Asp Leu Ile Gln Leu Val Glu Phe Tyr Gln Leu Asn Lys Gly Val
   145                 150                 155                 160
   Leu Pro Cys Lys Leu Lys His Tyr Cys Ala Arg Met Ala Val
                   165                 170
<210> 4
   <211> 186
   <212> PRT
   <213> Rattus sp.
<400> 4
   Gln Ala Arg Ser Ala Cys Ser Ser Gln Ser Val Ser Pro Met Arg Ser
   1               5                   10                  15
   Val Ser Glu Asn Ser Leu Val Ala Met Asp Phe Ser Gly Gln Lys Thr
               20                  25                  30
   Arg Val Ile Asp Asn Pro Thr Glu Ala Leu Ser Val Ala Val Glu Glu
           35                  40                  45
   Gly Leu Ala Trp Arg Lys Lys Gly Cys Leu Arg Leu Gly Asn His Gly
       50                  55                  60
   Ser Pro Thr Ala Pro Ser Gln Ser Ser Ala Val Asn Met Ala Leu His
   65                  70                  75                  80
   Arg Ser Gln Pro Trp Phe His His Arg Ile Ser Arg Asp Glu Ala Gln
                   85                  90                  95
   Gln Leu Ile Thr Arg Gln Gly Pro Val Asp Gly Val Phe Leu Val Arg
               100                 105                 110
   Asp Ser Gln Ser Asn Pro Arg Thr Phe Val Leu Ser Met Ser His Gly
           115                 120                 125
   Gln Lys Ile Lys His Phe Gln Ile Ile Pro Val Glu Asp Asp Gly Glu
       130                 135                 140
   Val Phe His Thr Leu Asp Asp Gly His Thr Lys Phe Thr Asp Leu Ile
   145                 150                 155                 160
   Gln Leu Val Glu Phe Tyr Gln Leu Asn Lys Gly Val Leu Pro Cys Lys
                   165                 170                 175
   Leu Lys His Tyr Cys Ala Arg Met Ala Val
               180                 185
<210> 5
   <211> 43
   <212> PRT
   <213> Homo sapiens
<400> 5
   Pro Met Arg Ser Ile Ser Glu Asn Ser Leu Val Ala Met Asp Phe Ser
   1               5                   10                  15
   Gly Gln Lys Ser Arg Val Ile Glu Asn Pro Thr Glu Ala Leu Ser Val
               20                  25                  30
   Ala Val Glu Glu Gly Leu Ala Trp Arg Lys Lys
           35                  40
<210> 6
   <211> 84
   <212> PRT
   <213> Homo sapiens
<400> 6
   Gln Gly Arg Ser Gly Cys Ser Ser Gln Ser Ile Ser Pro Met Arg Ser
   1               5                   10                  15
   Ile Ser Glu Asn Ser Leu Val Ala Met Asp Phe Ser Gly Gln Lys Ser
               20                  25                  30
   Arg Val Ile Glu Asn Pro Thr Glu Ala Leu Ser Val Ala Val Glu Glu
           35                  40                  45
   Gly Leu Ala Trp Arg Lys Lys Gly Cys Leu Arg Leu Gly Thr His Gly
       50                  55                  60
   Ser Pro Thr Ala Ser Ser Gln Ser Ser Ala Thr Asn Met Ala Ile His
   65                  70                  75                  80
   Arg Ser Gln Pro
<210> 7
   <211> 174
   <212> PRT
   <213> Homo sapiens
<400> 7
   Pro Met Arg Ser Ile Ser Glu Asn Ser Leu Val Ala Met Asp Phe Ser
    1               5                  10                  15
   Gly Gln Lys Ser Arg Val Ile Glu Asn Pro Thr Glu Ala Leu Ser Val
               20                  25                  30
   Ala Val Glu Glu Gly Leu Ala Trp Arg Lys Lys Gly Cys Leu Arg Leu
           35                  40                  45
   Gly Thr His Gly Ser Pro Thr Ala Ser Ser Gln Ser Ser Ala Thr Asn
       50                  55                  60
   Met Ala Ile His Arg Ser Gln Pro Trp Phe His His Lys Ile Ser Arg
   65                  70                  75                  80
   Asp Glu Ala Gln Arg Leu Ile Ile Gln Gln Gly Leu Val Asp Gly Val
                   85                  90                  95
   Phe Leu Val Arg Asp Ser Gln Ser Asn Pro Lys Thr Phe Val Leu Ser
               100                 105                 110
   Met Ser His Gly Gln Lys Ile Lys His Phe Gln Ile Ile Pro Val Glu
           115                 120                 125
   Asp Asp Gly Glu Met Phe His Thr Leu Asp Asp Gly His Thr Arg Phe
       130                 135                 140
   Thr Asp Leu Ile Gln Leu Val Glu Phe Tyr Gln Leu Asn Lys Gly Val
   145                 150                 155                 160
   Leu Pro Cys Lys Leu Lys His Tyr Cys Ala Arg Ile Ala Leu
                   165                 170
<210> 8
   <211> 186
   <212> PRT
   <213> Homo sapiens
<400> 8
   Gln Gly Arg Ser Gly Cys Ser Ser Gln Ser Ile Ser Pro Met Arg Ser
   1               5                   10                  15
   Ile Ser Glu Asn Ser Leu Val Ala Met Asp Phe Ser Gly Gln Lys Ser
               20                  25                  30
   Arg Val Ile Glu Asn Pro Thr Glu Ala Leu Ser Val Ala Val Glu Glu
           35                  40                  45
   Gly Leu Ala Trp Arg Lys Lys Gly Cys Leu Arg Leu Gly Thr His Gly
       50                  55                  60
   Ser Pro Thr Ala Ser Ser Gln Ser Ser Ala Thr Asn Met Ala Ile His
   65                  70                  75                  80
   Arg Ser Gln Pro Trp Phe His His Lys Ile Ser Arg Asp Glu Ala Gln
                   85                  90                  95
   Arg Leu Ile Ile Gln Gln Gly Leu Val Asp Gly Val Phe Leu Val Arg
               100                 105                 110
   Asp Ser Gln Ser Asn Pro Lys Thr Phe Val Leu Ser Met Ser His Gly
           115                 120                 125
   Gln Lys Ile Lys His Phe Gln Ile Ile Pro Val Glu Asp Asp Gly Glu
       130                 135                 140
   Met Phe His Thr Leu Asp Asp Gly His Thr Arg Phe Thr Asp Leu Ile
   145                 150                 155                 160
   Gln Leu Val Glu Phe Tyr Gln Leu Asn Lys Gly Val Leu Pro Cys Lys
                   165                 170                 175
   Leu Lys His Tyr Cys Ala Arg Ile Ala Leu
               180                 185
<210> 9
   <211> 43
   <212> PRT
   <213> mus muris
<400> 9
   Pro Met Arg Ser Val Ser Glu Asn Ser Leu Val Ala Met Asp Phe Ser
   1               5                   10                  15
   Gly Gln Ile Gly Arg Val Ile Asp Asn Pro Ala Glu Ala Gln Ser Ala
               20                  25                  30
   Ala Leu Glu Glu Gly His Ala Trp Arg Asn Gly
           35                  40
<210> 10
   <211> 82
   <212> PRT
   <213> mus muris
<400> 10
   Pro Gln Arg Lys Gly Leu Pro Pro Pro Phe Asn Ala Pro Met Arg Ser
    1               5                  10                  15
   Val Ser Glu Asn Ser Leu Val Ala Met Asp Phe Ser Gly Gln Ile Gly
               20                  25                  30
   Arg Val Ile Asp Asn Pro Ala Glu Ala Gln Ser Ala Ala Leu Glu Glu
           35                  40                  45
   Gly His Ala Trp Arg Asn Gly Ser Thr Arg Met Asn Ile Leu Ser Ser
       50                  55                  60
   Gln Ser Pro Leu His Pro Ser Thr Leu Asn Ala Val Ile His Arg Thr
   65                  70                  75                  80
   Gln His
<210> 11
   <211> 172
   <212> PRT
   <213> mus muris
<400> 11
   Pro Met Arg Ser Val Ser Glu Asn Ser Leu Val Ala Met Asp Phe Ser
    1               5                  10                  15
   Gly Gln Ile Gly Arg Val Ile Asp Asn Pro Ala Glu Ala Gln Ser Ala
               20                  25                  30
   Ala Leu Glu Glu Gly His Ala Trp Arg Asn Gly Ser Thr Arg Met Asn
           35                  40                  45
   Ile Leu Ser Ser Gln Ser Pro Leu His Pro Ser Thr Leu Asn Ala Val
       50                  55                  60
   Ile His Arg Thr Gln His Trp Phe His Gly Arg Ile Ser Arg Glu Glu
   65                  70                  75                  80
   Ser His Arg Ile Ile Lys Gln Gln Gly Leu Val Asp Gly Leu Phe Leu
                   85                  90                  95
   Leu Arg Asp Ser Gln Ser Asn Pro Lys Ala Phe Val Leu Thr Leu Cys
              100                 105                 110
   His His Gln Lys Ile Lys Asn Phe Gln Ile Leu Pro Cys Glu Asp Asp
   115 120 125
   Gly Gln Thr Phe Phe Thr Leu Asp Asp Gly Asn Thr Lys Phe Ser Asp
   130 135 140
   Leu Ile Gln Leu Val Asp Phe Tyr Gln Leu Asn Lys Gly Val Leu Pro
   145 150 155 160
   Cys Lys Leu Lys His His Cys Ile Arg Val Ala Leu
   165 170
<210> 12
   <211> 184
   <212> PRT
   <213> mus muris
<400> 12
   Pro Gln Arg Lys Gly Leu Pro Pro Pro Phe Asn Ala Pro Met Arg Ser
   1 5 10 15
   Val Ser Glu Asn Ser Leu Val Ala Met Asp Phe Ser Gly Gln Ile Gly
   20 25 30
   Arg Val Ile Asp Asn Pro Ala Glu Ala Gln Ser Ala Ala Leu Glu Glu
   35 40 45
   Gly His Ala Trp Arg Asn Gly Ser Thr Arg Met Asn Ile Leu Ser Ser
   50 55 60
   Gln Ser Pro Leu His Pro Ser Thr Leu Asn Ala Val Ile His Arg Thr
   65 70 75 80
   Gln His Trp Phe His Gly Arg Ile Ser Arg Glu Glu Ser His Arg Ile
   85 90 95
   Ile Lys Gln Gln Gly Leu Val Asp Gly Leu Phe Leu Leu Arg Asp Ser
   100 105 110
   Gln Ser Asn Pro Lys Ala Phe Val Leu Thr Leu Cys His His Gln Lys
   115 120 125
   Ile Lys Asn Phe Gln Ile Leu Pro Cys Glu Asp Asp Gly Gln Thr Phe
   130 135 140
   Phe Thr Leu Asp Asp Gly Asn Thr Lys Phe Ser Asp Leu Ile Gln Leu
   145 150 155 160
   Val Asp Phe Tyr Gln Leu Asn Lys Gly Val Leu Pro Cys Lys Leu Lys
   165 170 175
   His His Cys Ile Arg Val Ala Leu
   180
<210> 13
   <211> 43
   <212> PRT
   <213> Homo sapiens
<400> 13
   Pro Val Arg Ser Val Ser Glu Asn Ser Leu Val Ala Met Asp Phe Ser
   1               5                   10                  15
   Gly Gln Thr Gly Arg Val Ile Glu Asn Pro Ala Glu Ala Gln Ser Ala
               20                  25                  30
   Ala Leu Glu Glu Gly His Ala Trp Arg Lys Arg
           35                  40
<210> 14
   <211> 82
   <212> PRT
   <213> Homo sapiens
<400> 14
   Gln Gln Arg Lys Ala Leu Leu Ser Pro Phe Ser Thr Pro Val Arg Ser
   1               5                   10                  15
   Val Ser Glu Asn Ser Leu Val Ala Met Asp Phe Ser Gly Gln Thr Gly
               20                  25                  30
   Arg Val Ile Glu Asn Pro Ala Glu Ala Gln Ser Ala Ala Leu Glu Glu
           35                  40                  45
   Gly His Ala Trp Arg Lys Arg Ser Thr Arg Met Asn Ile Leu Gly Ser
       50                  55                  60
   Gln Ser Pro Leu His Pro Ser Thr Leu Ser Thr Val Ile His Arg Thr
   65                  70                  75                  80
   Gln His
<210> 15
   <211> 172
   <212> PRT
   <213> Homo sapiens
<400> 15
   Pro Val Arg Ser Val Ser Glu Asn Ser Leu Val Ala Met Asp Phe Ser
   1               5                   10                  15
   Gly Gln Thr Gly Arg Val Ile Glu Asn Pro Ala Glu Ala Gln Ser Ala
               20                  25                  30
   Ala Leu Glu Glu Gly His Ala Trp Arg Lys Arg Ser Thr Arg Met Asn
           35                  40                  45
   Ile Leu Gly Ser Gln Ser Pro Leu His Pro Ser Thr Leu Ser Thr Val
        50                  55                  60
   Ile His Arg Thr Gln His Trp Phe His Gly Arg Phe Ser Arg Glu Glu
    65                  70                  75                  80
   Ser His Arg Ile Ile Lys Gln Gln Gly Leu Val Asp Gly Leu Phe Leu
                    85                  90                  95
   Leu Arg Asp Ser Gln Ser Asn Pro Lys Ala Phe Val Leu Thr Leu Cys
               100                 105                 110
   His His Gln Lys Ile Lys Asn Phe Gln Ile Leu Pro Cys Glu Asp Asp
           115                 120                 125
   Gly Gln Thr Phe Phe Ser Leu Asp Asp Gly Asn Thr Lys Phe Ser Asp
       130                 135                 140
   Leu Ile Gln Leu Val Asp Phe Tyr Gln Leu Asn Lys Gly Val Leu Pro
   145                 150                 155                 160
   Cys Lys Leu Lys His His Cys Ile Arg Val Ala Leu
                   165                 170
<210> 16
   <211> 184
   <212> PRT
   <213> Homo sapiens
<400> 16
   Gln Gln Arg Lys Ala Leu Leu Ser Pro Phe Ser Thr Pro Val Arg Ser
    1               5                  10                  15
   Val Ser Glu Asn Ser Leu Val Ala Met Asp Phe Ser Gly Gln Thr Gly
               20                  25                  30
   Arg Val Ile Glu Asn Pro Ala Glu Ala Gln Ser Ala Ala Leu Glu Glu
           35                  40                  45
   Gly His Ala Trp Arg Lys Arg Ser Thr Arg Met Asn Ile Leu Gly Ser
       50                  55                  60
   Gln Ser Pro Leu His Pro Ser Thr Leu Ser Thr Val Ile His Arg Thr
   65                  70                  75                  80
   Gln His Trp Phe His Gly Arg Phe Ser Arg Glu Glu Ser His Arg Ile
                   85                  90                  95
   Ile Lys Gln Gln Gly Leu Val Asp Gly Leu Phe Leu Leu Arg Asp Ser
               100                 105                 110
   Gln Ser Asn Pro Lys Ala Phe Val Leu Thr Leu Cys His His Gln Lys
           115                 120                 125
   Ile Lys Asn Phe Gln Ile Leu Pro Cys Glu Asp Asp Gly Gln Thr Phe
       130                 135                 140
   Phe Ser Leu Asp Asp Gly Asn Thr Lys Phe Ser Asp Leu Ile Gln Leu
   145                 150                 155                 160
   Val Asp Phe Tyr Gln Leu Asn Lys Gly Val Leu Pro Cys Lys Leu Lys
                   165                 170                 175
   His His Cys Ile Arg Val Ala Leu
               180
<210> 17
   <211> 43
   <212> PRT
   <213> Rattus sp.
<400> 17
   Pro Leu Arg Ser Val Ser Asp Asn Thr Leu Val Ala Met Asp Phe Ser
    1               5                  10                  15
   Gly His Ala Gly Arg Val Ile Asp Asn Pro Arg Glu Ala Leu Ser Ala
               20                  25                  30
   Ala Met Glu Glu Ala Gln Ala Trp Arg Lys Lys
           35                  40
<210> 18
   <211> 80
   <212> PRT
   <213> Rattus sp.
<400> 18
   Ser Arg His Leu Arg Leu Ser Tyr Leu Gly Ser Pro Pro Leu Arg Ser
    1               5                  10                  15
   Val Ser Asp Asn Thr Leu Val Ala Met Asp Phe Ser Gly His Ala Gly
               20                  25                  30
   Arg Val Ile Asp Asn Pro Arg Glu Ala Leu Ser Ala Ala Met Glu Glu
           35                  40                  45
   Ala Gln Ala Trp Arg Lys Lys Thr Asn His Arg Leu Ser Leu Pro Thr
       50                  55                  60
   Thr Cys Ser Gly Ser Ser Leu Ser Ala Ala Ile His Arg Thr Gln Pro
   65                  70                  75                  80
<210> 19
   <211> 170
   <212> PRT
   <213> Rattus sp.
<400> 19
   Pro Leu Arg Ser Val Ser Asp Asn Thr Leu Val Ala Met Asp Phe Ser
    1               5                  10                  15
   Gly His Ala Gly Arg Val Ile Asp Asn Pro Arg Glu Ala Leu Ser Ala
               20                  25                  30
   Ala Met Glu Glu Ala Gln Ala Trp Arg Lys Lys Thr Asn His Arg Leu
           35                  40                  45
   Ser Leu Pro Thr Thr Cys Ser Gly Ser Ser Leu Ser Ala Ala Ile His
       50                  55                  60
   Arg Thr Gln Pro Trp Phe His Gly Arg Ile Ser Arg Glu Glu Ser Gln
   65                  70                  75                  80
   Arg Leu Ile Gly Gln Gln Gly Leu Val Asp Gly Val Phe Leu Val Arg
                   85                  90                  95
   Glu Ser Gln Arg Asn Pro Gln Gly Phe Val Leu Ser Leu Cys His Leu
               100                 105                 110
   Gln Lys Val Lys His Tyr Leu Ile Leu Pro Ser Glu Asp Glu Gly Cys
           115                 120                 125
   Leu Tyr Phe Ser Met Asp Glu Gly Gln Thr Arg Phe Thr Asp Leu Leu
       130                 135                 140
   Gln Leu Val Glu Phe His Gln Leu Asn Arg Gly Ile Leu Pro Cys Leu
   145                 150                 155                 160
   Leu Arg His Cys Cys Ala Arg Val Ala Leu
                   165                 170
<210> 20
   <211> 182
   <212> PRT
   <213> Rattus sp.
<400> 20
   Ser Arg His Leu Arg Leu Ser Tyr Leu Gly Ser Pro Pro Leu Arg Ser
    1               5                  10                  15
   Val Ser Asp Asn Thr Leu Val Ala Met Asp Phe Ser Gly His Ala Gly
               20                  25                  30
   Arg Val Ile Asp Asn Pro Arg Glu Ala Leu Ser Ala Ala Met Glu Glu
           35                  40                  45
   Ala Gln Ala Trp Arg Lys Lys Thr Asn His Arg Leu Ser Leu Pro Thr
       50                  55                  60
   Thr Cys Ser Gly Ser Ser Leu Ser Ala Ala Ile His Arg Thr Gln Pro
   65                  70                  75                  80
   Trp Phe His Gly Arg Ile Ser Arg Glu Glu Ser Gln Arg Leu Ile Gly
                   85                  90                  95
   Gln Gln Gly Leu Val Asp Gly Val Phe Leu Val Arg Glu Ser Gln Arg
               100                 105                 110
   Asn Pro Gln Gly Phe Val Leu Ser Leu Cys His Leu Gln Lys Val Lys
           115                 120                 125
   His Tyr Leu Ile Leu Pro Ser Glu Asp Glu Gly Cys Leu Tyr Phe Ser
       130                 135                 140
   Met Asp Glu Gly Gln Thr Arg Phe Thr Asp Leu Leu Gln Leu Val Glu
   145                 150                 155                 160
   Phe His Gln Leu Asn Arg Gly Ile Leu Pro Cys Leu Leu Arg His Cys
                   165                 170                 175
   Cys Ala Arg Val Ala Leu
               180
<210> 21
   <211> 43
   <212> PRT
   <213> Homo sapiens
<400> 21
   Pro Leu Arg Ser Ala Ser Asp Asn Thr Leu Val Ala Met Asp Phe Ser
   1               5                   10                  15
   Gly His Ala Gly Arg Val Ile Glu Asn Pro Arg Glu Ala Leu Ser Val
               20                  25                  30
   Ala Leu Glu Glu Ala Gln Ala Trp Arg Lys Lys
           35                  40
<210> 22
   <211> 80
   <212> PRT
   <213> Homo sapiens
<400> 22
   Ser Arg His Leu His Pro Ser Cys Leu Gly Ser Pro Pro Leu Arg Ser
    1               5                  10                  15
   Ala Ser Asp Asn Thr Leu Val Ala Met Asp Phe Ser Gly His Ala Gly
               20                  25                  30
   Arg Val Ile Glu Asn Pro Arg Glu Ala Leu Ser Val Ala Leu Glu Glu
           35                  40                  45
   Ala Gln Ala Trp Arg Lys Lys Thr Asn His Arg Leu Ser Leu Pro Met
       50                  55                  60
   Pro Ala Ser Gly Thr Ser Leu Ser Ala Ala Ile His Arg Thr Gln Leu
   65                  70                  75                  80
<210> 23
   <211> 170
   <212> PRT
   <213> Homo sapiens
<400> 23
   Pro Leu Arg Ser Ala Ser Asp Asn Thr Leu Val Ala Met Asp Phe Ser
    1               5                  10                  15
   Gly His Ala Gly Arg Val Ile Glu Asn Pro Arg Glu Ala Leu Ser Val
               20                  25                  30
   Ala Leu Glu Glu Ala Gln Ala Trp Arg Lys Lys Thr Asn His Arg Leu
           35                  40                  45
   Ser Leu Pro Met Pro Ala Ser Gly Thr Ser Leu Ser Ala Ala Ile His
       50                  55                  60
   Arg Thr Gln Leu Trp Phe His Gly Arg Ile Ser Arg Glu Glu Ser Gln
   65                  70                  75                  80
   Arg Leu Ile Gly Gln Gln Gly Leu Val Asp Gly Leu Phe Leu Val Arg
                   85                  90                  95
   Glu Ser Gln Arg Asn Pro Gln Gly Phe Val Leu Ser Leu Cys His Leu
               100                 105                 110
   Gln Lys Val Lys His Tyr Leu Ile Leu Pro Ser Glu Glu Glu Gly Arg
           115                 120                 125
   Leu Tyr Phe Ser Met Asp Asp Gly Gln Thr Arg Phe Thr Asp Leu Leu
       130                 135                 140
   Gln Leu Val Glu Phe His Gln Leu Asn Arg Gly Ile Leu Pro Cys Leu
   145                 150                 155                 160
   Leu Arg His Cys Cys Thr Arg Val Ala Leu
                   165                 170
<210> 24
   <211> 182
   <212> PRT
   <213> Homo sapiens
<400> 24
   Ser Arg His Leu His Pro Ser Cys Leu Gly Ser Pro Pro Leu Arg Ser
    1               5                   10                  15
   Ala Ser Asp Asn Thr Leu Val Ala Met Asp Phe Ser Gly His Ala Gly
               20                  25                  30
   Arg Val Ile Glu Asn Pro Arg Glu Ala Leu Ser Val Ala Leu Glu Glu
           35                  40                  45
   Ala Gln Ala Trp Arg Lys Lys Thr Asn His Arg Leu Ser Leu Pro Met
       50                  55                  60
   Pro Ala Ser Gly Thr Ser Leu Ser Ala Ala Ile His Arg Thr Gln Leu
   65                  70                  75                  80
   Trp Phe His Gly Arg Ile Ser Arg Glu Glu Ser Gln Arg Leu Ile Gly
                   85                  90                  95
   Gln Gln Gly Leu Val Asp Gly Leu Phe Leu Val Arg Glu Ser Gln Arg
               100                 105                 110
   Asn Pro Gln Gly Phe Val Leu Ser Leu Cys His Leu Gln Lys Val Lys
           115                 120                 125
   His Tyr Leu Ile Leu Pro Ser Glu Glu Glu Gly Arg Leu Tyr Phe Ser
       130                 135                 140
   Met Asp Asp Gly Gln Thr Arg Phe Thr Asp Leu Leu Gln Leu Val Glu
   145                 150                 155                 160
   Phe His Gln Leu Asn Arg Gly Ile Leu Pro Cys Leu Leu Arg His Cys
                   165                 170                 175
   Cys Thr Arg Val Ala Leu
               180
<210> 25
   <211> 43
   <212> PRT
   <213> mus muris
<400> 25
   Pro Leu Arg Ser Val Ser Asp Asn Thr Leu Val Ala Met Asp Phe Ser
   1 5 10 15
   Gly His Ala Gly Arg Val Ile Asp Asn Pro Arg Glu Ala Leu Ser Ala
   20 25 30
   Ala Met Glu Glu Ala Gln Ala Trp Arg Lys Lys
   35 40
<210> 26
   <211> 80
   <212> PRT
   <213> mus muris
<400> 26
   Ser Arg His Leu Arg Leu Ser Tyr Leu Gly Ser Pro Pro Leu Arg Ser
   1 5 10 15
   Val Ser Asp Asn Thr Leu Val Ala Met Asp Phe Ser Gly His Ala Gly
   20 25 30
   Arg Val Ile Asp Asn Pro Arg Glu Ala Leu Ser Ala Ala Met Glu Glu
   35 40 45
   Ala Gln Ala Trp Arg Lys Lys Thr Asn His Arg Leu Ser Leu Pro Thr
   50 55 60
   Thr Cys Ser Gly Ser Ser Leu Ser Ala Ala Ile His Arg Thr Gln Pro
   65 70 75 80
<210> 27
   <211> 170
   <212> PRT
   <213> mus muris
<400> 27
   Pro Leu Arg Ser Val Ser Asp Asn Thr Leu Val Ala Met Asp Phe Ser
   1               5                   10                  15
   Gly His Ala Gly Arg Val Ile Asp Asn Pro Arg Glu Ala Leu Ser Ala
               20                  25                  30
   Ala Met Glu Glu Ala Gln Ala Trp Arg Lys Lys Thr Asn His Arg Leu
           35                  40                  45
   Ser Leu Pro Thr Thr Cys Ser Gly Ser Ser Leu Ser Ala Ala Ile His
       50                  55                  60
   Arg Thr Gln Pro Trp Phe His Gly Arg Ile Ser Arg Glu Glu Ser Gln
   65                  70                  75                  80
   Arg Leu Ile Gly Gln Gln Gly Leu Val Asp Gly Val Phe Leu Val Arg
                   85                  90                  95
   Glu Ser Gln Arg Asn Pro Gln Gly Phe Val Leu Ser Leu Cys His Leu
               100                 105                 110
   Gln Lys Val Lys His Tyr Leu Ile Leu Pro Ser Glu Asp Glu Gly Cys
           115                 120                 125
   Leu Tyr Phe Ser Met Asp Glu Gly Gln Thr Arg Phe Thr Asp Leu Leu
       130                 135                 140
   Gln Leu Val Glu Phe His Gln Leu Asn Arg Gly Ile Leu Pro Cys Leu
   145                 150                 155                 160
   Leu Arg His Cys Cys Ala Arg Val Ala Leu
                   165                 170
<210> 28
   <211> 182
   <212> PRT
   <213> mus muris.
<400> 28
   Ser Arg His Leu Arg Leu Ser Tyr Leu Gly Ser Pro Pro Leu Arg Ser
    1               5                  10                  15
   Val Ser Asp Asn Thr Leu Val Ala Met Asp Phe Ser Gly His Ala Gly
               20                  25                  30
   Arg Val Ile Asp Asn Pro Arg Glu Ala Leu Ser Ala Ala Met Glu Glu
           35                  40                  45
   Ala Gln Ala Trp Arg Lys Lys Thr Asn His Arg Leu Ser Leu Pro Thr
       50                  55                  60
   Thr Cys Ser Gly Ser Ser Leu Ser Ala Ala Ile His Arg Thr Gln Pro
   65                  70                  75                  80
   Trp Phe His Gly Arg Ile Ser Arg Glu Glu Ser Gln Arg Leu Ile Gly
                   85                  90                  95
   Gln Gln Gly Leu Val Asp Gly Val Phe Leu Val Arg Glu Ser Gln Arg
               100                 105                 110
   Asn Pro Gln Gly Phe Val Leu Ser Leu Cys His Leu Gln Lys Val Lys
           115                 120                 125
   His Tyr Leu Ile Leu Pro Ser Glu Asp Glu Gly Cys Leu Tyr Phe Ser
       130                 135                 140
   Met Asp Glu Gly Gln Thr Arg Phe Thr Asp Leu Leu Gln Leu Val Glu
   145                 150                 155                 160
   Phe His Gln Leu Asn Arg Gly Ile Leu Pro Cys Leu Leu Arg His Cys
                   165                 170                 175
   Cys Ala Arg Val Ala Leu
               180